# EUROPÄISCHE PATENTANMELDUNG

(11) **EP 1 288 838 A2**
(43) Veröffentlichungstag der Anmeldung: **05.03.2003**
(21) Anmeldenummer: 02018603.7
(22) Anmeldetag: 19.08.2002
(51) Int. Cl.: G06F 19/00

(54) **Patienteninformationssystem zur Erläuterung von medizinischen Befunden**

(30) Priorität: 30.08.2001 DE 10142341
(71) Anmelder: SIEMENS AKTIENGESELLSCHAFT, 80333 München (DE)
(72) Erfinder: Abraham-Fuchs, Klaus, 91058 Erlangen (DE); Bieger, Johannes, Dr., 80638 München (DE); Hengerer, Arne, Dr., 91053 Erlangen (DE); Rumpel, Eva, Dr., 91052 Erlangen (DE); Tietze, Daniel, 91080 Spardorf (DE)

(57) **Zusammenfassung**

Patienteninformationssystem zur patientenorientierten interaktiven Erläuterung von medizinischen Befunden, wobei ein mit einer für den Patienten zugänglichen Eingabeeinrichtung versehenes Expertensystem, welches die in einem eingegebenen medizinischen Befund enthaltenen Informationen, basierend auf einem in ihm hinterlegten Regelwerk, entsprechend ebenfalls eingegebenen individuellen Informationen über den Patienten interpretiert und erläutert, insbesondere den durch medizinische Fachausdrücke unverständlichen Befund in eine verständlichere, ausführlichere Erläuterungen enthaltene Alltagssprache übersetzt.

## Beschreibung

Die Erfindung bezieht sich auf ein Patienteninformationssystem zur patientenorientierten, interaktiven Erläuterung von medizinischen Befunden.

Ob anlässlich einer akuten Erkrankung oder einer Vorsorgemaßnahme werden an einem Patienten Messungen und Untersuchungen durchgeführt, deren Ergebnisse in den ärztlichen Befunden dokumentiert werden und Grundlage für die darauf aufbauende Diagnose und anschließende Behandlung bilden. Auf Nachfrage oder auch routinemäßig (Privatpatienten) erhalten Patienten eine Kopie ihrer Befunde wie z.B. der Blutwerte oder Krankenbefunde, sei es in Form eines Laborberichts oder als Kopie eines Arztbriefes. Jedoch sind diese Dokumente für einen medizinischen Laien oft kaum verständlich und für eine eingehende Erläuterung durch den Arzt fehlt meist die Zeit. Diese Situation ist für viele Patienten sehr unbefriedigend, da sie gerne besser über die Untersuchungsergebnisse und deren Konsequenzen informiert wären. Da ein informierter Patient aber auch ein motivierterer Patient ist, vergeben sich Ärzte, aber auch Versicherungen, an dieser Stelle häufig eine Chance, über eine bessere Einbindung und Aufklärung der Patienten eine höhere Compliance zu erreichen.

Bisher waren interessierte Patienten darauf angewiesen, mittels Printmedien, wie z.B. Nachschlagewerken, populärwissenschaftliche Veröffentlichungen und einführende Fachliteratur, sich kundig zu machen. Diese Informationen sind jedoch allgemein gehalten und es erfordert große Mühe und Zeitaufwand, die für den eigenen Fall relevanten Informationen herauszufiltern. In letzter Zeit bieten mehrere Dienstleister einen Service an, bei dem ärztliches Personal via E-mail oder am Telefon Patientenfragen beantwortet. Dieses Verfahren ist aber sehr personalaufwändig und dementsprechend teuer und stellt letztendlich überhaupt keine Verbesserung gegenüber einer teuren ausführlichen Information durch den behandelnden Arzt dar. Angebote zu mäßigen Preisen wie sie via E-mail bereits angeboten werden, gehen jedoch nicht weiter auf die persönlichen Voraussetzungen des Patienten, wie z.B. seinen Bildungsstand, ein, wobei stets noch hinzu kommt, dass die off-line Recherche einen Zeitverzug mit sich bringt.

Aus der DE 690 30 664 T2 ist bereits ein Expertensystem bekannt, bei dem es aber um eine computergestützte Diagnose geht, das heißt das System soll aufgrund von Eingaben über Befindlichkeiten des Patienten mögliche ursächliche Krankheiten erkennen und diagnostizieren können. Das im Rahmen dieser Anmeldung gestellte Problem liegt aber nicht in der Erstellung einer Diagnose, sondern in der Interpretation einer bereits gefundenen Diagnose, die durch ihre relativ unverständliche medizinische Befundformulierung für einen Patienten nicht verständlich ist.

Das gleiche gilt auch für ein Verfahren und eine Vorrichtung zur Online- Interpretation medizinischer Daten entsprechend der DE 100 65 580 A1. Hier geht es um die Aufbereitung medizinischer Daten wiederum zum Zwecke einer computergestützten Diagnose, nicht aber zur Interpretation eines für einen Laien unverständlichen medizinischen Befundes.

Der Erfindung liegt daher die Aufgabe zugrunde, ein Patienteninformationssystem zur patientenorientierten interaktiven Erläuterung von medizinischen Befunden zu schaffen, das individuell auf den Patienten, auch speziell auf seinen Vorkenntnisstand, abgestimmte Interpretationen und Erläuterungen zu ärztlichen Befunden erarbeitet.

Zur Lösung dieser Aufgabe ist erfindungsgemäß ein, mit einer für den Patienten zugänglichen Eingabevorrichtung versehenes Expertensystem vorgesehen, welches die in einem eingegebenen medizinischen Befund enthaltenen Informationen, basierend auf einem in ihm hinterlegten Regelwerk, entsprechend ebenfalls eingegebenen individuellen Informationen über den Patienten z. B. den Vorbildungsgrad und das Sprachverständnis oder die Krankengeschichte des Patienten, interpretiert und erläutert, insbesondere den durch medizinische Fachausdrücke unverständlichen Befund in eine verständlichere, ausführlichere Erläuterungen enthaltende Alltagssprache übersetzt.

Die Eingabeeinrichtung ist dabei im einfachsten Falle eine Tastatur oder, vorzugsweise auch zusätzlich dazu, ein Scanner, um beispielsweise einen Arztbrief oder Laborbericht direkt und ohne umständliches Eingeben in die Tastatur einlesen zu können. Falls der Befund bereits in digitalisierter Form vorliegt, kann er auch direkt- beispielsweise über ein link in das Expertensystem eingegeben, bzw. von ihm angefordert werden.

Im einfachsten Fall gibt der Patient neben dem Befund auch individuelle Patientendaten zusätzlich ein, wie beispielsweise seinen Kenntnisstand, momentanen Gesundheitszustand oder frühere Krankheiten. Um gerade diese individuellen Patientendaten erfassen zu können ist bevorzugt das Expertensystem mit einem selbständigen Zugriff zur Eingabeeinrichtung versehen, um im Bedarfsfall interpretierungsrelevante Rückfragen zu stellen. Es kann also nach Eingabe des Befundes vom Expertensystem ein kleiner Fragenkatalog an die Eingabevorrichtung zurückgeschickt werden, der vom Patienten bearbeitet werden muss, damit das Expertensystem die speziellen vorstehend angesprochenen individuellen Patientendaten gezielt abfragen kann.

In Weiterbildung der Erfindung soll das Expertensystem Zugriff zu einer zentralen oder dezentralen elektronischen Patientenakte besitzen, in der diese individuellen Patientendaten weitestgehend bereits enthalten sind, so dass eine nochmalige Eingabe durch den anfragenden Patienten gar nicht nötig ist. Speziell in diesem Fall muss dann aber die Eingabeeinrichtung zweckmäßigerweise mit einem mittels Pin, Chipkarte oder dergleichen gesicherten Patientenidentifizierungssystem versehen sein, damit die Autorisierung und die Zugriffsberechtigung des anfragenden Patienten zu seiner elektronischen Patientenakte gesichert ist. Das Expertensystem braucht dabei nicht alle für eine Erläuterung und Interpretation eines Befundes notwendigen Informationen direkt eingespeichert haben, sondern es genügt, wenn es neben einem grundsätzlichen Interpretationsregelwerk mit lexikalischen Datenbanken in Verbindung steht, wie z.B. Krankheitslexikon, Lexikon der medizinischen Fachbegriffe, Lexikon der diagnoseverfahren und deren Aussagekraft oder dergleichen.

Handelt es sich bei den Ausgangsdaten, also bei Befunddaten, die über die Eingabevorrichtung eingegeben werden, nicht um stark strukturiertes Datenmaterial, wie zum Beispiel Laborwerte oder einen auf Textbausteinen basierenden Befund oder Arztbrief, sondern um einen freien Fließtext, so muss zunächst eine Textinterpretation erfolgen. Hierzu ist erfindungsgemäß ein, vorzugsweise in das Expertensystem integriertes spezielles Textinterpretationssystem zur Aufbereitung unstrukturierter Befunde vorgesehen.

Die Entscheidung, welche Fragen dem Patienten gestellt werden müssen, trifft das Expertensystem basierend auf den in ihm hinterlegten Regelwerk. Die eingehenden Daten versetzen das Expertensystem in die Lage, das Wissensbedürfnis des Nutzers zu klassifizieren und somit individuell zugeschnittene Informationen bereitzustellen.

Das Expertensystem liefert dem Benutzer dann als Ergebnis eine auf sein Sprachniveau abgestimmte textuelle Interpretation der Laborwerte, des Befundes oder Arztbriefes sowie evtl. nützliche Hintergrundinformatinen, wie z.B. die Erläuterung der Fachtermini, Erläuterung der Grenz- und Schwellwerte, Vor- und Nachteile von Behandlungsalternativen, Links zu weiterführenden Informationen wie Selbsthilfegruppen und Second Opinion etc..

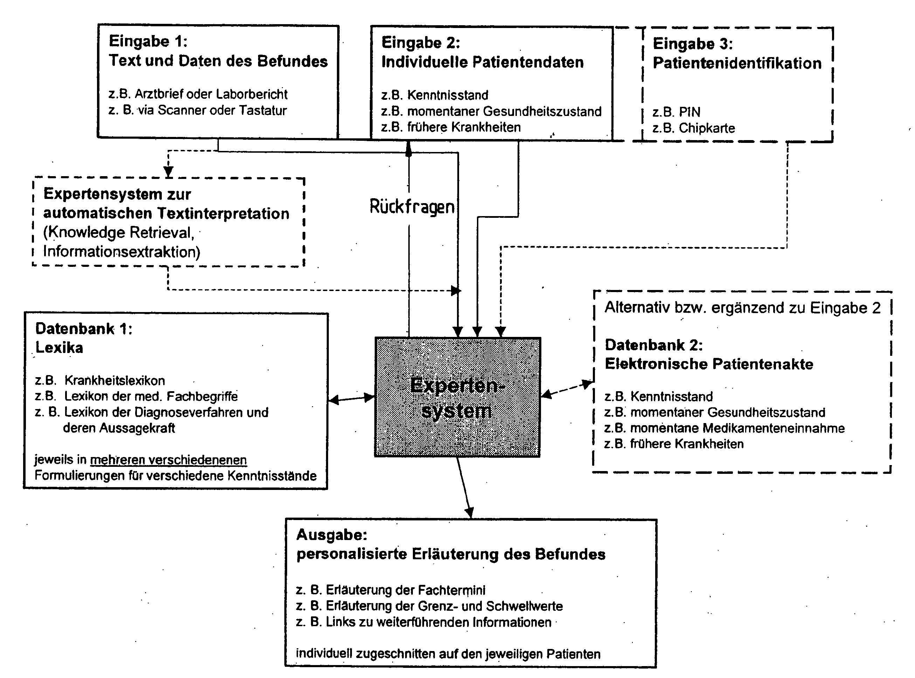

Weitere Vorteile, Merkmale und Einzelheiten der Erfindung ergeben sich aus der nachfolgenden Beschreibung eines Ausführungsbeispiels sowie anhand der Zeichnung.

Ein Expertensystem mit einem darin hinterlegten Regelwerk zur Interpretierung medizinischer Befunde ist mit einer Eingabeeinrichtung verbunden, mittels derer der Patient über eine Eingabeeinrichtung zunächst den Text und die Daten des Befundes in das Expertensystem eingeben kann. Hierzu kann entweder ein Scanner oder eine Tastatur vorgesehen sein, wobei beispielsweise zur Eingabe eines Bluttests und seiner Ergebnisse der Patient zum Beispiel über das Internet das passende Formular wählt und die bei ihm erhobenen Werte in dieses Formular einträgt.

Als Eingabe können daneben die individuellen Patientendaten eingegeben werden, zum Beispiel sein Kenntnisstand, der momentane Gesundheitszustand oder frühere Krankheiten, wobei diese Eingabe individueller Patientendaten bevorzugt in direkter Rückkopplung mit dem Expertensystem erfolgt, das die Entscheidung trifft, welche dieser angegebenen individuellen Patientendaten es speziell für seine Interpretation des medizinischen Befundes benötigt. Das Expertensystem fragt also bei der Eingabeeinrichtung an und der Patient gibt die entsprechenden Erläuterungen ab.

Das Expertensystem ist mit einer Datenbank verbunden, das eine Vielzahl von unterschiedlichen medizinischen Lexika enthalten kann wie zum Beispiel Krankheitslexikon, Lexikon der medizinischen Fachbegriffe, Lexikon der Diagnoseverfahren und deren Aussagekraft, wodurch unter anderem für jeden Befundbegriff mehrere verschiedene Formulierungen für verschiedene Kenntnisstände zur Verfügung stehen, die das Expertensystem basierend auf den individuellen Patientendaten dahingehend ausnutzen kann, dass es bei der Ausgabe, nämlich der personalisierten Erläuterung des Befundes, auf diese individuellen Patientendaten, speziell seinen Kenntnisstand, auch Rücksicht nehmen kann. Weiß nämlich das Expertensystem, dass der anfragende Patient ein Laie mit Volksschulvorbildung ist, so kann er ganz andere Begriffe zur Erläuterung wählen und insbesondere auch Fachtermini speziell erläutern, die er nicht erläutern wird, wenn er erfährt, dass der anfragende Patient gewisse medizinische Vorbildung oder einen sonstigen höheren Bildungsgrad besitzt.

Mit besonderem Vorteil ist das Expertensystem mit einer zweiten Datenbank verbunden, die eine zentrale oder dezentrale elektronische Patientenakte enthält. In dieser sind all die individuellen Patientendaten, die ansonsten über die Eingabe der individuellen Patientendaten vom Patienten selbst eingegeben werden müssen, bereits vorhanden und häufig sogar in einer wesentlich detaillierteren Form. Insbesondere erspart dies dem Patienten häufig peinliche Rückfragen über seinen Bildungszustand oder dergleichen, da dies ja in der elektronischen Patientenakte vermerkt ist.

Speziell dann, wenn das Expertensystem mit der elektronischen Patientenakte verbunden ist, bzw. Rückgriff auf diese nehmen kann, ist in der Eingabeeinrichtung ein Patientenidentifikationssystem erforderlich, das über eine PIN-Nummer oder eine Chipkarte, ein Fingerabdruck oder Retina-Erkennungssystem oder dergleichen abgesichert ist, so dass sichergestellt ist, dass der anfragende Patient auch tatsächlich der ist, dessen elektronische Patientenakte vom Expertensystem mit ausgewertet werden soll.

Bei Eingabe des eingangs angesprochenen Bluttests als zu interpretierender medizinischer Befund und dem speziell aus der Patientendatenbank ermittelten Bildungsstand des Nutzers (zum Beispiel: Verständnis biologischer Zusammenhänge vorhanden) sowie besonders zu beachtender Umstände (Nutzer ist Diabetiker) kann das System schließlich dem Patienten individuell angepasste Informationen unter anderem über die Aussagekraft der einzelnen Blutparameter, die zugehörigen Schwellwerte, die Bedenklichkeit der in diesem Fall gemessenen Werte, die Behandlungsmöglichkeiten sowie Adress- und Literaturverweise als Ausgabe liefern.

In Fällen, in denen der Befund oder Arztbrief nicht so strukturiert ist, wie dies bei den Daten eines Blutbildes der Fall ist, wenn also beispielsweise ein freier Fließtext als Befund vorhanden ist, kann zusätzlich ein Textinterpretationsprogramm zur Aufbereitung dieser umstrukturierten Befunde vorgesehen sein, wobei dieses Textinterpretationsprogramm, das in der Figur als Expertensystem zur automatischen Textinterpretation bezeichnet ist, bevorzugt direkt in das Expertensystem eingegliedert ist. Gerade in diesem Fall ist auch die Möglichkeit der Rückfragen des Expertensystems beim eingebenden Patienten bedeutungsvoll, da in einem Befund beispielsweise von einem Tumor der Klassifizierung 17 die Rede sein kann, der zunächst auch für das erläuternde Expertensystem nicht ganz klar ist. Um sich hier Sicherheit zu verschaffen, kann das Expertensystem beim Patienten rückfragen, ob bei ihm Krebs diagnostiziert worden ist, je nachdem, ob der Patient mit ja oder nein antwortet, ist die Tumorklassifizierung in anderer Weise auszulegen.

## Patentansprüche

1. Patienteninformationssystem zur patientenorientierten interaktiven Erläuterung von medizinischen Befunden, **gekennzeichnet durch** ein mit einer für den Patienten zugänglichen Eingabeeinrichtung versehenes Expertensystem, welches die in einem eingegebenen medizinischen Befund enthaltenen Informationen, basierend auf einem in ihm hinterlegten Regelwerk, entsprechend ebenfalls eingegebenen individuellen Informationen über den Patienten interpretiert und erläutert, insbesondere den **durch** medizinische Fachausdrücke unverständlichen Befund in eine verständlichere, ausführlichere Erläuterungen enthaltene Alltagssprache übersetzt.

2. Patienteninformationssystem nach Anspruch 1, **dadurch gekennzeichnet, dass** die individuellen Informationen den Vorbildungsgrad und/oder das Sprachverständnis und/oder die Krankengeschichte des Patienten umfassen.

3. Patienteninformationssystem nach Anspruch 1 oder 2, **dadurch gekennzeichnet, dass** die Eingabeeinrichtung eine Tastatur und/oder einen Scanner umfasst.

4. Patienteninformationssystem nach einem der Ansprüche 1 bis 3, **dadurch gekennzeichnet, dass** die Eingabeeinrichtung ein mittels PIN, Chipkarte oder dergleichen gesichertes Patientenidentifizierungssystem enthält.

5. Patienteninformationssystem nach einem der Ansprüche 1 bis 4, **dadurch gekennzeichnet, dass** das Expertensystem einen selbständigen Zugriff zur Eingabeeinrichtung besitzt, um im Bedarfsfall interpretierungsrelevante Rückfragen zu stellen.

6. Patienteninformationssystem nach einem der Ansprüche 1 bis 5, **dadurch gekennzeichnet, dass** das Expertensystem Zugriff zu einer zentralen oder dezentralen elektronischen Patientenakte hat.

7. Patienteninformationssystem nach einem der Ansprüche 1 bis 6, **dadurch gekennzeichnet, dass** das Expertensystem mit lexikalischen Datenbanken wie zum Beispiel einem Krankheitslexikon, Lexikon der medizinischen Fachbegriffe, Lexikon der Diagnoseverfahren und deren Aussagekraft oder dergleichen verbunden ist.

8. Patienteninformationssystem nach einem der Ansprüche 1 bis 7, **gekennzeichnet durch** ein, vorzugsweise in das Expertensystem integriertes, Textinterpretationssystem zur Aufbereitung unstrukturierter Befunde.
